# EUROPEAN PATENT SPECIFICATION

(11) **EP 1 641 397 B1**
(45) Date of publication and mention of the grant of the patent: **16.12.2009**
(21) Application number: 04749140.2
(22) Date of filing: 06.07.2004
(51) Int. Cl.: A61B 6/03

(54) **SCANNING-BASED DETECTION OF IONIZING RADIATION FOR TOMOSYNTHESIS**
AUF SCANNING BASIERENDER NACHWEIS VON IONISIERENDER STRAHLUNG FÜR DIE TOMOSYNTHESE
DETECTION PAR BALAYAGE D'UN RAYONNEMENT IONISANT POUR TOMOSYNTHESE

(30) Priority: 08.07.2003 SE 0302022; 08.01.2004 SE 0400010
(43) Date of publication of application: 05.04.2006
(73) Proprietor: XCounter AB, 182 33 Danderyd (SE)
(72) Inventor: ULLBERG, Christer, S-191 34 Sollentuna (SE); SOKOLOV, Skiff, S-181 35 Lidingö (SE); FRANCKE, Tom, S-191 44 Sollentuna (SE)
(74) Representative: Fritzon, Rolf
(86) International application number: PCT/SE2004/001104
(87) International publication number: WO 2005/002443

(56) References cited:
- WO-A1-01/59480
- GB-A- 2 070 883
- US-A- 5 032 990
- US-B1- 6 320 929

## Description

### FIELD OF THE INVENTION

The invention relates generally to scanning-based apparatuses and methods for obtaining tomosynthesis data for examination of an object.

### BACKGROUND,OF THE INVENTION AND RELATED ART

An X-ray medical diagnostic method such as mammography is a low-dose procedure that creates one or more images of a part of a patient such as a breast thereof, which is to be examined, e.g. for detection of early stages of cancer.

The mammography diagnostic procedure generally includes obtaining two images of each of the patient's breasts, one from above and one from the side. A physician or radiologist then reviews the images of the breast, i.e., mammograms, to identify any breast cancer.

While this procedure is one of the best methods of detecting early forms of breast cancer, it is still possible for the detection of breast cancer to be missed by a physician or radiologist reviewing the mammograms. For example, breast cancer may be missed by being obscured by radiographically dense, fibroglandular breast tissue.

Tomosynthesis imaging, in which a plurality of images is acquired at different angles, has been studied in an effort to detect early forms of breast cancer. By combining the plurality of images, it is possible to reconstruct any plane in the breast being imaged that is parallel to the detector. The higher number of images is utilized, the better image quality in the reconstructed tomosynthesis images is obtained.

Document US-B1-6 320 929 discloses a scanning-based tomography apparatus comprising a divergent radiation source for emitting radiation around an axis of symmetry, a radiation detector comprising a stack of line detectors, each line detector being directed towards the divergent radiation source to allow a ray bundle of said radiation that propagates in a respective one of a plurality of different angles to enter the line detector, and a device for rotating the radiation source and the radiation detector while the object to be scanned is linearly moved in a direction essentially orthogonal to the axis of symmetry.

Document GB-A-2 070 883 discloses an apparatus for obtaining tomosynthesis data of an object, the apparatus comprising a stack of detectors which is directed towards the radiation source at a certain angle, and in which source and detector moves linearly relative to the object.

Further, various line detectors for detecting ionizing radiation are known in the art. While such detectors provide for instantaneous one-dimensional imaging, two-dimensional imaging can only be performed by means of scanning the line detector, and optionally the radiation source, in a direction traverse to the one-dimensional detector array. To use such a detector in tomosynthesis, wherein a plurality of images has to be acquired at different angles would be very time consuming.

### SUMMARY OF THE INVENTION

A main object of the invention is therefore to provide a scanning-based apparatus and a method, respectively, for obtaining tomosynthesis data of an object at a higher speed than what is obtainable by using scanning-based apparatuses and methods of the prior art. The apparatus of the invention is defined in claim 1 and the method of the invention is defined in claim 14.

In this respect there is a particular object to provide such an apparatus and such a method, which are capable of instantaneously recording, by means multiple one-dimensional detectors, multiple one-dimensional images of the object, and, by means of scanning, multiple two-dimensional images of the object, where each of the one-dimensional images of the object is recorded at a different angle.

A further object of the invention is to provide such an apparatus and such a method, which are capable of recording, by means of scanning a number of one-dimensional detectors over the object, a number of two-dimensional images of the object, where each of the two-dimensional images of the object is recorded at a different angle, and where the number of the two-dimensional images is higher than the number of one-dimensional detectors.

A still further object of the invention is to provide such an apparatus and such a method, which are uncomplicated and can produce high-quality two-dimensional tomosynthesis .images with high spatial resolution, high signal-to-noise ratio, high dynamic range, high image contrast, and low noise from overlaying tissue.

A yet further object of the invention is to provide such an apparatus and such a method, which are reliable, accurate, and inexpensive.

These objects, among others, are attained by apparatuses and methods as claimed in the appended claims.

The inventors have found that by providing a divergent radiation source emitting radiation centered around an axis of symmetry, and a radiation detector comprising a stack of line detectors, each being directed towards the divergent radiation source to allow a ray bundle of the radiation that propagates in a respective one of a plurality of different angles to enter the line detector after having been transmitted through an object to be examined, and moving the radiation source and the radiation detector relative the object linearly in a direction orthogonal to the axis of symmetry, while each of the line detectors records line images of radiation as transmitted through the object in a respective one of the different angles, a plurality of two-dimensional images can be formed, where each two-dimensional image is formed from a plurality of line images as recorded by a single one of the line detectors.

The movement is made a length sufficient for scanning each of the line detectors across the entire object.

Thus, a plurality of two-dimensional images at different angles are produced in a single scan, which reduces the detection time by a factor corresponding to the number of two-dimensional images produced.

Preferably, a device is provided for rotating the radiation detector around an axis of rotation being orthogonal to the axis of symmetry, wherein the line detectors, after the rotation, are each directed towards the divergent radiation source to allow a ray bundle of the radiation that propagates in a respective one of a further plurality of different angles to enter the line detector, and the device for moving is further arranged to repeat the linear movement of the divergent radiation source and the radiation detector relative the object, while each of the line detectors is adapted to record a further plurality of line images of radiation as transmitted through the object in a respective one of the further plurality of different angles.

The data from the apparatus is excellent to be used in tomosynthesis or laminographic imaging.

The line detectors uses are preferably, but not exclusively, gaseous-based parallel plate detectors. Other line detectors that may be used include scintillator-based arrays, CCD arrays, TFT- and CMOS-based detectors, liquid detectors, and diode arrays, e.g. PIN-diode arrays with edge-on, near edge-on or perpendicular incidence of X-rays. A collimator structure may be arranged in front of the detectors to partly reject scattered X-rays.

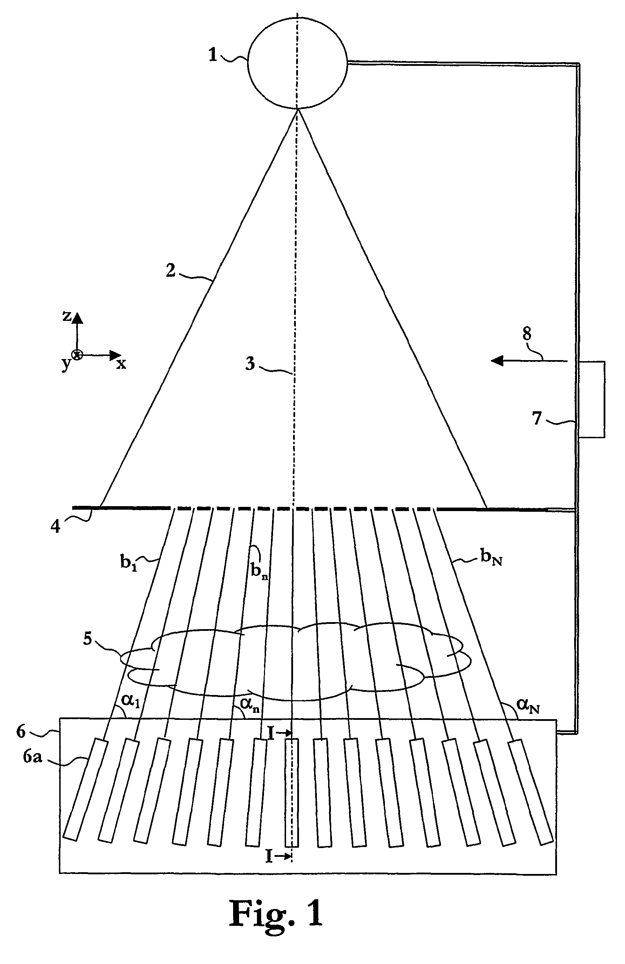

Further characteristics of the invention and advantages thereof, will be evident from the detailed description of preferred embodiments of the present invention given hereinafter and the accompanying Figs. 1-4, which are given by way of illustration only and thus, are not limitative of the present invention.

### BRIEF DESCRIPTION OF THE DRAWINGS

Fig. 1 illustrates schematically, in a top view, an apparatus for obtaining tomosynthesis data for x-ray examination of an object according to a preferred embodiment of the present invention.
Figs. 2a-c illustrate each schematically, in a top view, a particular X-ray bundle as it traverses the examination object during a first scanning movement by the apparatus of Fig. 1.
Figs. 3a-c illustrate each schematically, in a top view, a particular X-ray bundle as it traverses the examination object during a second scanning movement by the apparatus of Fig. 1.
Fig. 4 illustrates schematically a cross-sectional view of a line detector of Fig. 1 as taken along the line I-I.

### DESCRIPTION OF PREFERRED EMBODIMENTS

The apparatus of Fig. 1 comprises a divergent X-ray source 1, which produces X-rays 2 centered around an axis of symmetry 3 (parallel with the z axis), a collimator 4, a radiation detector 6, and a device 7 for rigidly connecting the X-ray source 1, the collimator 4, and the radiation detector 6 to each other and moving the X-ray source 1, the collimator 4, and the radiation detector 6 essentially linearly in direction 8 (typically parallel with the x axis) essentially orthogonal to the axis of symmetry 3 to scan an object 5, which is to be examined.

The radiation detector 6 comprises a stack of line detectors 6a, each being directed towards the divergent radiation source 1 to allow a respective ray bundle b1, ..., bₙ, ..., b_{N} of the radiation 2 that propagates in a respective one of a plurality of different angles α₁. ...., αₙ, ..., α_{N}, with respect to the front surface of the radiation detector 6 to enter the respective line detector 6a. The line detectors 6a are extending in the y direction to record line images extending in the y direction.

The collimator 4 may be a thin foil of e.g. tungsten with narrow radiation transparent slits etched away, the number of which corresponds to the number of line detectors 6a of the radiation detector 6. The slits are aligned with the line detectors 6a so that X-rays passing through the slits of the collimator 4 will reach the detector units 6a, i.e. as the respective ray bundles b₁, ..., bₙ, ..., b_{N}. The collimator 4, which is optional, prevents radiation, which is not directed directly towards the line detectors 6a, from impinging on the object 5, thereby reducing the radiation dose to the object. This is advantageous in all applications where the object is a human or an animal, or parts thereof.

During scanning the device 7 moves the radiation source 1, the collimator 4, and the radiation detector 6 relative the object 5 in a linear manner parallel with the front of the radiation detector as being indicated by arrow 8, while each of the line detectors 6a records a plurality of line images of radiation as transmitted through the object 5 in a respective one of the different angles α₁, ..., αₙ, ..., α_{N}.

The scanning of the object 5 is performed a length, which is sufficiently large so that *each one* of the line detectors 6a can be scanned across the entire object of interest to obtain, for each of the line detectors 6a, a two-dimensional image of radiation as transmitted through the object 5 in a respective one of the different angles α₁, ..., αₙ, ..., α_{N}.

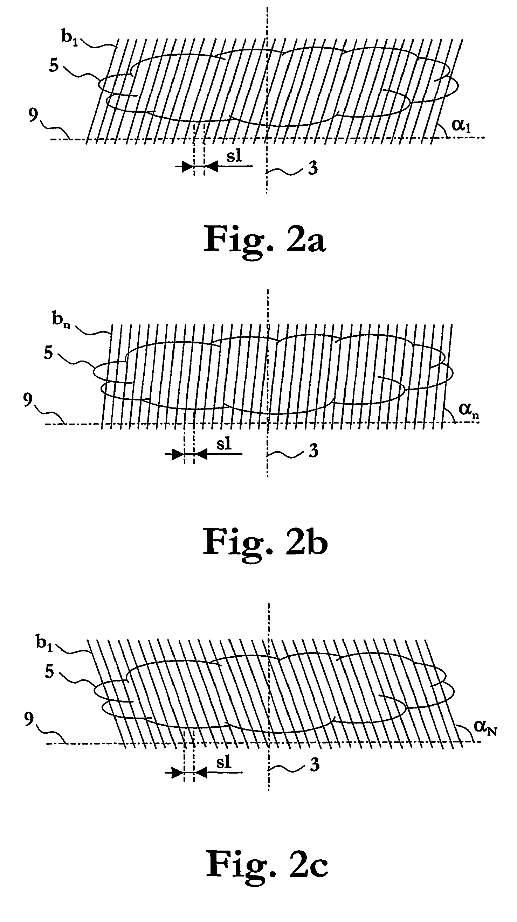

In Figs. 2a-c three different X-ray bundles b₁, bₙ, and b_{N} are schematically illustrated as they traverse the examination object 5 during scanning by the apparatus of Fig. 1. Reference numeral 9 indicates a plane parallel with the x axis, which coincides with the scanning direction 8 and with the front of the radiation detector 6.

As can be seen in Figs. 2a-c each line detector/X-ray bundle pair produces a complete two-dimensional image at a distinct one of the different angles. Fig. 2a illustrates the formation of a two-dimensional image of radiation transmitted through the object at an angle α₁, Fig. 2b illustrates the formation of a two-dimensional image of radiation transmitted through the same object, but at an angle αₙ, and Fig. 2c illustrates the formation of a similar two-dimensional image, but at an angle α_{N}.

Preferably, the different angles are distributed over an angular range α_{N}-α₁ of at least 5°, preferably at least 10°, and most preferably at least 15° depending on the application or kind of examination in order to obtain high-quality tomosynthesis data for examination of the object. The number of line detectors 6a in the stack of line detectors is at least 3, preferably at least 10, and most preferably at least 25 depending on the number of images recorded at different angles, which is required during the examination.

The scanning step, in Figs. 2a-c denoted by sl, depends on the spatial resolution of the two-dimensional images formed from the one-dimensional recordings. Typically, the scanning step sl can be about 10-500 microns, and the individual detecting elements of each of the line detectors can be of similar size.

Advantageously, the device 7 for performing the scanning movement, or other device (not illustrated), is capable of rotating the radiation source 1, the collimator 4, and the radiation detector 6 an angle Δ around an axis of rotation passing e.g. through the radiation source 1 or other point, and being orthogonal to the axis of symmetry 3, and preferably parallel with the y axis. The angle Δ is preferably smaller than the difference between two one adjacent ones of the different angles α₁, ..., αₙ, ..., α_{N}.

The radiation source 1 may, however, be kept still during the rotation if the line detectors 6a, after the rotation, are still within the solid angle of radiation as emitted by the radiation source 1.

The device 7 for moving then repeats the linear movement of the radiation source 1, the collimator 4, and the radiation detector 6 relative the object 5 in a second scan, while each of the line detectors records further multiple line images of radiation as transmitted through the object 5 in a respective one of the further different angles α₁+Δ, ..., αₙ+Δ, ..., α_{N}+Δ.

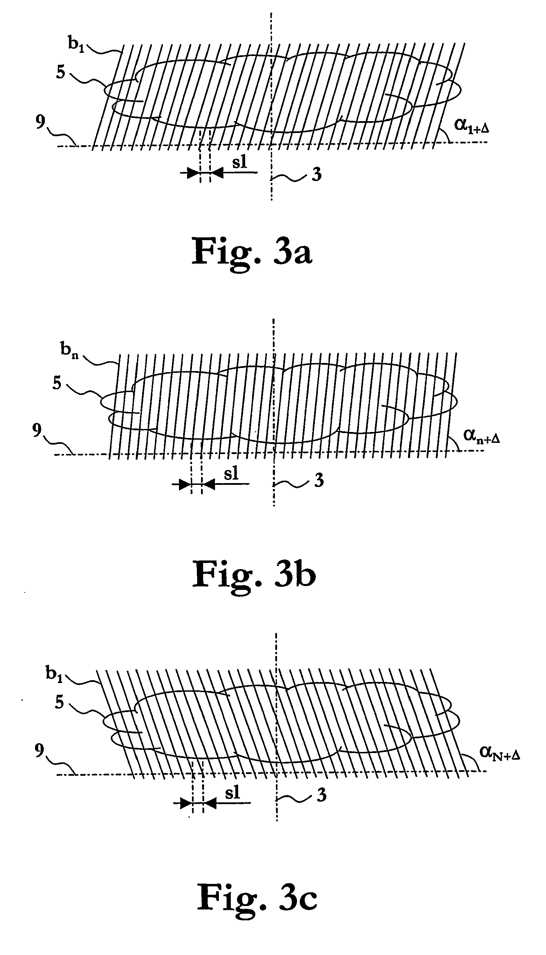

In Figs. 3a-c three different X-ray bundles b₁, bₙ, and b_{N} are schematically illustrated as they traverse the examination object 5 during the second scanning by the apparatus of Fig. 1. Reference numeral 9 indicates as in Fig. 2 a plane parallel with the x axis, which here slightly deviates from the scanning direction 8 and from the front of the radiation detector 2 due to the rotation.

As can be seen in Figs. 3a-c each line detector/X-ray bundle pair produces a complete two-dimensional image at a distinct one of the different angles. Fig. 3a illustrates the formation of a two-dimensional image of radiation transmitted through the object at an angle α₁+Δ, Fig. 3b illustrates the formation of a two-dimensional image of radiation transmitted through the same object, but at an angle αₙ+Δ, and Fig. 3c illustrates the formation of a similar two-dimensional image, but at an angle α_{N}+Δ.

Thus, two linear scans with a slight rotation therein between provide for the formation of 2N two-dimensional images at the different angles α₁, α₁+Δ, ..., αₙ, αₙ+Δ, ..., α_{N}, α_{N}+Δ. Similarly, the rotation and linear scanning may be repeated P times to obtain PxN two-dimensional images. In such a manner a large number of images at different angles may be obtained by using a limited number of line detectors. Hereby, a low cost radiation detector can be provided to the cost of a prolonged scanning and examination time. The total radiation dose to the object 5 during the examination has, however, not necessarily to be increased.

Alternatively, or additionally, rotation may be performed around an axis of rotation, which is parallel with the x axis, between linear scans of the above-described kind.

The more images at different angles are provided, the smaller is the noise from overlaying objects in the reconstructed tomosynthesis image.

It shall be noted that the present invention is applicable to any kind of examination employing tomosynthesis or laminographic imaging, including e.g. mammography examination and other soft tissue examinations.

A preferred line detector for use in the present invention is a gaseous-based parallel plate detector, preferably provided with an electron avalanche amplifier. Such a gaseous-based parallel plate detector is an ionization detector, wherein electrons freed as a result of ionization by ionizing radiation are accelerated in a direction essentially perpendicular to the direction of the radiation.

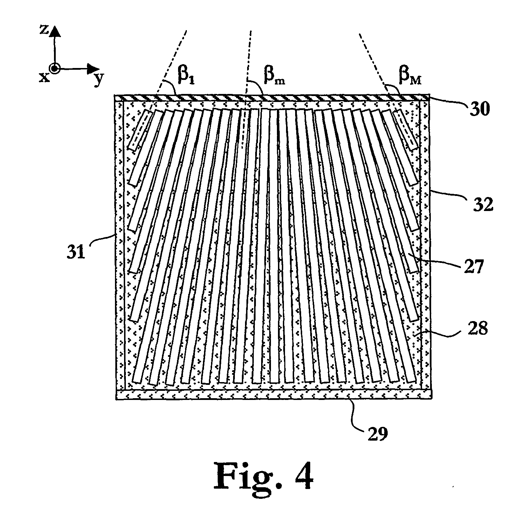

A cross-sectional view of a line detector of Fig. 1 as taken along the line I-I is schematically illustrated in Fig. 4.

The line detector comprises a window 30 for entry of a ray bundle, and a row of elongated individual conductive detector elements or strips 27 arranged on a dielectric substrate 28. Preferably, the elements or strips 27, which each is capable of separately detecting incident radiation photons, also constitute an anode of the line detector to attract the electrons released during ionization of the ionizable gas in the line detector. Preferably, the dielectric substrate 28 and the window 30 define, together with sidewalls 29, 31, 32 and a non-illustrated dielectric cathode substrate, a gas-tight confinement capable of being filled with the ionizable gas. Alternatively, the line detector is arranged within an external gas-tight casing (not illustrated).

Note that the individual conductive detector/anode elements 27 are arranged side by side in a row parallel with the y direction, and define a respective angle β₁, ..., βₘ, ..., β_{M} with respect to the xz plane so that all the detector/anode elements 27 point towards the X-ray source 1 to avoid any parallax errors caused by the divergent radiation. As a result different detector/anode elements 27 detect different angular portions β₁, ..., βₘ, ..., β_{M} of the ray bundle entered into the line detector.

For further details regarding such kind of gaseous-based line detectors for use in the present invention, reference is made to the following U.S. Patents by Tom Francke et al. and assigned to XCounter AB of Sweden: Nos. 6,546,070; 6,522,722; 6,518,578; 6,118,125; 6,373,065; 6,337,482; 6,385,282; 6,414,317; 6,476,397; and 6,477,223.

It shall particularly be pointed out that such kind of detector is very efficient in preventing Compton scattered radiation from being detected. This property is of outermost importance to obtain high-quality tomosynthesis data.

The distance between the parallel plates, i.e. electrodes, of the line detector may be below about 2 mm, preferably below about 1 mm, more preferably below about 0.5 mm, and most preferably between about 0.1 mm and 0.5 mm. XCounter AB has recently begun to verify the Compton scattering rejection characteristics of the line detector experimentally and good contrast has been observed using a wide X-ray spectrum of high energy X-rays, at which conditions a conventional detector system would not be capable to see any structure at all. It is believed that the above-depicted gaseous-based line detector discriminates more than 99% of the scattered photons; and by proper design it is assumed that about 99.9% or more of the scattered photons can be prevented from being detected.

It shall, nevertheless, be realized that any other type of detector may be used in the present invention. Such line detectors include scintillator-based arrays, CCD arrays, TFT- and CMOS-based detectors, liquid detectors, and solid-state detectors such as one-dimensional PIN-diode arrays with edge-on, near edge-on or perpendicular incidence of X-rays, possibly with a collimator structure in front to partly reject scattered X-rays.

It shall further be noted that that the device 7 for rigidly connecting the X-ray source 1, the collimator 4, and the radiation detector 6 may be exchanged for separate devices (not illustrated) for the X-ray source 1, the collimator 4, and the radiation detector 6, which may be controlled electronically to obtain synchronous movements of the separate devices to obtain similar scanning movement. Yet alternatively, the apparatus of Fig. 1 can be modified so the object 5 is moved during scanning, while the radiation source 1, the collimator 4, and the radiation detector 6 are kept at rest.

It shall still further be noted that instead of performing linear scans for each rotation, the linear scanning may be performed stepwise, and at each such linear scanning step measurements are made for different rotations. The result is identical, but the measurements are performed in different order.

It shall yet further be noted that in an alternative embodiment of the present invention the linear scanning is performed in the y direction and the rotation between the linear scannings is performed around an axis parallel with the x axis. This calls for a very short distance between the line detectors in the stack since one detector strip from each line detector provides the instantaneous one-dimensional image. One complete scan in the y direction involves that each of the detector strips of each of the line detectors is moved across the complete object. The strips of the plurality of line detectors, which are inclined to the xz plane with the angle β₁, record, during the scan, one two-dimensional image, the adjacent strips record another two-dimensional image at a different angle, etc. After the first scan the detector is rotated an angle +Δ around an axis parallel with the x axis. During the second scan, the strips of the plurality of line detectors are inclined to the xz plane with β₁+Δ, ..., βₘ+Δ, ..., β_{M}+Δ. Thus, two linear scans with a slight rotation therein between provide for the formation of 2M two-dimensional images at the different angles β₁, β₁+Δ, ,.., βₘ, βₘ+Δ, ..., β_{M}, β_{M}+Δ. Similarly, the rotation and linear scanning may be repeated P times to obtain PxM two-dimensional images.

## Claims

1. A scanning-based apparatus for obtaining tomosynthesis data of an object (5) comprising:
- a divergent radiation source (1) for emitting radiation (2) centered around an axis of symmetry (3);
- a radiation detector (6) comprising a stack of line detectors (6a), each being directed towards the divergent radiation source so that a ray bundle (b₁, ..., bₙ, ..., b_{N}) of said radiation propagates in a respective one of a plurality of different angles (α₁, ..., αₙ, ..., α_{N}) to enter the line detector;
- an object area arranged in the radiation path between said divergent radiation source and said radiation detector for housing said object; and
- a device (7) for moving said divergent radiation source and said radiation detector relative said object essentially linearly in a direction (8) essentially orthogonal to said axis of symmetry while each of said line detectors (6a) records a plurality of line images of radiation transmitted through said object in said respective one of said plurality of different angles, wherein
- said device (7) for moving is adapted to move said divergent radiation source and said radiation detector relative said object a length which allows scanning each of said line detectors across the entire object to obtain, for each of said line detectors, a two-dimensional image of the radiation transmitted through said object in said respective one of said plurality of different angles.

2. The apparatus of claim 1 comprising
- a device for rotating said radiation detector (6) an angle (Δ) around an axis of rotation orthogonal to said axis of symmetry (3), the line detectors (6a) being, after said rotation, each directed towards the divergent radiation source to allow a ray bundle of said radiation that propagates in a respective one of a further plurality of different angles (α₁+Δ, ..., αₙ+Δ, ..., α_{N}+Δ) to enter the line detector, wherein
- said device (7) for moving is further arranged to repeat the essentially linear movement of said divergent radiation source and said radiation detector relative said object, while each of said line detectors is adapted to record a further plurality of line images of radiation as transmitted through said object in a respective one of said further plurality of different angles (α₁+Δ, ..., αₙ+Δ, ..., α_{N}+Δ).

3. The apparatus of claim 2 wherein said axis of rotation passes through said divergent radiation source (1).

4. The apparatus of claim 2 or 3 wherein
- said device for rotating is adapted to repeatedly rotate said radiation detector (6) around said axis of rotation, the line detectors (6a) being, after each of said rotations, each directed towards the divergent radiation source to allow a ray bundle of said radiation that propagates in a respective angle to enter the line detector; and
- said device (7) for moving is adapted, after each of said rotations, to repeat the essentially linear movement of said divergent radiation source and said radiation detector relative said object, while each of said line detectors is adapted to record line images of radiation transmitted through said object in said respective angle.

5. The apparatus of any of claims 2-4 wherein said line detectors are oriented to detect line images extending in a direction (y) essentially orthogonal to said axis of symmetry (3) and essentially orthogonal to the direction (8) for moving said divergent radiation source and said radiation detector relative said object.

6. The apparatus of claim 5 wherein said direction (y) in which said line images extend is parallel with said axis of rotation.

7. The apparatus of any of claims 2-4 wherein
- said line detectors are oriented to detect line images extending in a direction (y) essentially orthogonal to said axis of symmetry (3) and essentially orthogonal to the direction (x) for moving said divergent radiation source and said radiation detector relative said object; and
- said direction (y) in which said line images extend is essentially orthogonal to said axis of rotation (x).

8. The apparatus of any of claims 1-7 wherein said plurality of different angles are distributed over an angular range (α_{N}-α₁) of at least 5°, preferably at least 10°, and most preferably at least 15°.

9. The apparatus of any of claims 1-8 wherein the number of line detectors (6a) in said stack of line detectors is at least 3, preferably at least 10, and most preferably at least 25.

10. The apparatus of any of claims 1-9 wherein
- said divergent radiation source is an X-ray source (1); and
- said line detectors are each a gaseous-based ionization detector (6a), wherein electrons freed as a result of ionization by a respective ray bundle are accelerated in a direction essentially perpendicular to the direction of that ray bundle.

11. The apparatus of claim 10 wherein said gaseous-based ionization detector is an electron avalanche detector (6a).

12. The apparatus of any of claims 1-11 wherein said line detectors are each any of a diode array, a scintillator-based array, a CCD array, a TFT- or CMOS-based detector, or a liquid detector (6a).

13. The apparatus of any of claims 1-12 comprising a collimator (4) arranged in the radiation path between said radiation source and said object area, said collimator preventing radiation, which is not directed towards said line detectors, from impinging on said object, thereby reducing the radiation dose to said object.

14. A scanning-based method for obtaining tomosynthesis data of an object (5) comprising the following steps:
using a divergent radiation source (1) which emits radiation (2) centered around an axis of symmetry (3); and a radiation detector (6) comprising a stack of line detectors (6a), each being directed towards the divergent radiation source so that a ray bundle (b₁, ..., bₙ, ..., b_{N}) of said radiation propagates in a respective one of a plurality of different angles (α₁, ..., αₙ, ..., α_{N}) to enter the line detector;
- arranging said object in the radiation path between said divergent radiation source and said radiation detector; and
- moving said divergent radiation source and said radiation detector relative said object essentially linearly in a direction (8) essentially orthogonal to said axis of symmetry, while a plurality of line images of radiation transmitted through said object in said respective one of said plurality of different angles is recorded by each of said line detectors, wherein said movement is performed a length which allows scanning each of said line detectors across the entire object to obtain, for each of said line detectors, a two-dimensional image of the radiation transmitted through said object in said respective one of said plurality of different angles.

15. The method of claim 14 comprising the further steps of:
- rotating said radiation detector (6) an angle (Δ) around an axis of rotation orthogonal to said axis of symmetry (3), the line detectors (6a) being, after said rotation, each directed towards the divergent radiation source to allow a ray bundle of said radiation that propagates in a respective one of a further plurality of different angles (α₁+Δ, ..., αₙ+Δ, ..., α_{N}+Δ) to enter the line detector; and
- repeating the essentially linear movement of said divergent radiation source and said radiation detector relative said object, while each of said line detectors is adapted to record a further plurality of line images of radiation as transmitted through said object in a respective one of said further plurality of different angles (α₁+Δ, ..., αₙ+Δ, ..., α_{N}+Δ).

16. The method of claim 15 wherein said axis of rotation passes through said divergent radiation source (1).

## Patentansprüche

1. Scanner-basierte Vorrichtung zur Gewinnung von Tomosynthese-Daten eines Objekts (5), umfassend:
- eine Quelle divergenter Strahlung (1) zur Emission von Strahlung (2), zentriert um eine Symmetrieachse (3);
- einen Strahlungsdetektor (6) umfassend einen Stapel ("stack") an Liniendetektoren (6a), jeder ausgerichtet auf die Quelle divergenter Strahlung, so dass ein Strahlenbündel (b₁, ..., bₙ, ..., b_{N}) dieser Strahlung das in einem zugehörigen aus einer Vielzahl von unterschiedlichen Winkeln (α₁, ..., aₙ, ..., α_{N}) propagiert, in den Liniendetektor einfällt;
- einen Objektbereich, angesiedelt im Strahlungsweg zwischen der Quelle divergenter Strahlung und dem Strahlungsdetektor, um das Objekt aufzunehmen; und
- eine Einrichtung (7), um die Quelle divergenter Strahlung und den Strahlungsdetektor relativ zu dem Objekt im Wesentlichen linear in einer im Wesentlichen orthogonalen Richtung (8) zur genannten Symmetrieachse zu bewegen, während jeder dieser Liniendetektoren (6a) eine Vielzahl an Linien-Bildern der Strahlung, die durch das Objekt hindurch in dem zugehörigen Winkel aus einer Vielzahl von unterschiedlichen Winkeln transmittiert werden, aufnimmt, wobei
- diese Einrichtung zur Bewegung (7) dafür ausgelegt ist, die Quelle divergenter Strahlung und den Strahlungsdetektor relativ zu dem Objekt über eine Länge zu bewegen, die jedem der Liniendetektoren das Scannen durch das gesamte Objekt hindurch ermöglicht, um für jeden der Liniendetektoren ein zweidimensionales Bild der durch das Objekt in dem zugehörigen aus der Vielzahl von unterschiedlichen Winkeln transmittierten Strahlung zu erhalten.

2. Vorrichtung nach Anspruch 1, umfassend
- eine Einrichtung zum Drehen des Strahlungsdetektors (6) um einen Winkel (Δ) um eine Rotationsachse orthogonal zur Symmetrieachse (3), wobei die Liniendetektoren (6a) nach dieser Rotation jeweils auf die Quelle divergierender Strahlung hin ausgerichtet sind, und es erlauben, dass ein Strahlenbündel dieser Strahlung, das in einem zugehörigen Winkel aus einer weiteren Vielzahl an unterschiedlichen Winkeln (α₁ + Δ, ..., αₙ + Δ, ..., α_{N} + Δ) propagiert, in den Liniendetektor einfallen kann, wobei
- die Einrichtung (7) zur Bewegung außerdem so angeordnet ist, dass die im Wesentlichen lineare Bewegung der Quelle divergenter Strahlung und des Strahlungsdetektors relativ zum Objekt wiederholt wird und dabei jeder der Liniendetektoren dafür ausgelegt ist, eine weitere Vielzahl von Linienbildern der Strahlung, wie durch das Objekt in einem zugehörigen der weiteren Vielzahl an unterschiedlichen Winkeln (α₁ + Δ, ..., αₙ + Δ, ..., α_{N} + Δ) transmittiert, aufzuzeichnen.

3. Vorrichtung nach Anspruch 2, wobei die Rotationsachse durch die Quelle divergenter Strahlung (1) hindurchführt.

4. Vorrichtung nach Anspruch 2 oder 3, wobei
- die Einrichtung zur Rotation so ausgelegt ist, um den Strahlungsdetektor (6) wiederholt um die Rotationsachse zu drehen, wobei die Liniendetektoren (6a) nach jeder dieser Rotationen jeweils auf die divergierende Strahlungsquelle hin ausgerichtet sind, so dass ein Strahlenbündel der Strahlung, das in einem zugehörigen Winkel propagiert, in den Liniendetektor einfallen kann; und
- die Einrichtung (7) zur Bewegung so ausgelegt ist, um nach jeder dieser Rotationen die im Wesentlichen lineare Bewegung der Quelle divergenter Strahlung und des Strahlungsdetektors relativ zum Objekt zu wiederholen, während jeder der Liniendetektoren in der Lage ist, die Linienbilder der Strahlung aufzuzeichnen, die im zugehörigen Winkel durch das Objekt transmittiert werden.

5. Vorrichtung nach einem der Ansprüche 2 bis 4, wobei die Liniendetektoren ausgerichtet sind für die Detektion von Linienbilder, die sich in einer Richtung (y), im Wesentlichen orthogonal zur Symmetrieachse (3) und im Wesentlichen orthogonal zu der Richtung (8) ausdehnen, in der die Quelle divergenter Strahlung und der Strahlungsdetektor relativ zu dem Objekt bewegt werden.

6. Vorrichtung nach Anspruch 5, wobei die Richtung (y), in der sich die Linienbilder ausdehnen, parallel zur Rotationsachse ist.

7. Vorrichtung nach einem der Ansprüche 2 bis 4, wobei
- die Liniendetektoren ausgerichtet sind für die Detektion von Linienbildern, die sich in eine Richtung (y) im Wesentlichen orthogonal zur Symmetrieachse (3) und im Wesentlichen orthogonal zu der Richtung (x) ausdehnen, in der die Quelle divergenter Strahlung und der Strahlungsdetektor relativ zu dem Objekt bewegt werden können; und
- die Richtung (y), in der sich die Linienbilder ausdehnen, im Wesentlichen orthogonal zur Rotationsachse (x) ist.

8. Vorrichtung nach einem der Ansprüche 1 bis 7, wobei diese Vielzahl an unterschiedlichen Winkeln über einen Winkelbereich (α_{N} bis α₁) von mindestens 5°, vorzugsweise mindestens 10° und am meisten bevorzugt mindestens 15° verteilt sind.

9. Vorrichtung nach einem der Ansprüche 1 bis 8, wobei die Anzahl an Liniendetektoren (6a) in dem Stapel von Liniendetektoren mindestens 3, vorzugsweise mindestens 10, und am meisten bevorzugt mindestens 25 beträgt.

10. Vorrichtung nach einem der Ansprüche 1 bis 9, wobei
- die Quelle divergenter Strahlung eine Röntgenstrahlenquelle (1) ist; und
- die Liniendetektoren jeweils gas-basierte lonisationsdetektoren (6a) sind, wobei durch lonisation mittels eines respektiven Strahlenbündels freigesetzte Elektronen in eine Richtung im Wesentlichen senkrecht zu der Richtung des Strahlenbündels beschleunigt werden.

11. Vorrichtung nach Anspruch 10, wobei der gas-basierte lonisationsdetektor ein Elektronenlawinen-Detektor (6a) ist.

12. Vorrichtung nach einem der Ansprüche 1 bis 11, wobei die Liniendetektoren jeweils beliebig sind, aus einer Diodenanordnung, einer szintillationsbasierten Anordnung, einer CCD-Anordnung, einem TFT- oder CMOSbasierten Detektor oder einem Flüssigdetektor (6a).

13. Vorrichtung nach einem der Ansprüche 1 bis 12, umfassend einen Kollimator (4), der in dem Strahlungsweg zwischen der Strahlungsquelle und dem Objektbereich angeordnet ist, wobei dieser Kollimator verhindert, dass Strahlung auf das Objekt trifft, die nicht gegen die Liniendetektoren gerichtet ist, wodurch die Strahlendosis für das Objekt verringert wird.

14. Scanning-basiertes Verfahren um von einem Objekt (5) Tomosynthese-Daten zu erhalten, umfassend die folgenden Schritte:
Benutzen einer Quelle divergenter Strahlung (1), die Strahlung (2) emittiert, die um eine Symmetrieachse (3) zentriert ist; und einen Strahlungsdetektor (6), umfassend einen Stapel ("stack") von Liniendetektoren (6a), jeder gerichtet auf die Quelle divergenter Strahlung, so dass ein Strahlenbündel (b₁, ..., bₙ, ..., b_{N}) dieser Strahlung in einem zugehörigen Winkel aus einer Vielzahl von unterschiedlichen Winkeln (α₁, ..., αₙ, ..., α_{N}) propagiert und in den Liniendetektor einfällt;
- Anordnen des Objekts in dem Strahlungsweg zwischen der Quelle divergenter Strahlung und dem Strahlungsdetektor; und
- Bewegen der divergenten Strahlungsquelle und des Strahlungsdetektors relativ zu dem Objekt im Wesentlichen linear in einer Richtung (8) im Wesentlichen orthogonal zu der Symmetrieachse, während eine Vielzahl von Linienbildern der Strahlung, die durch das Objekt in dem zugehörigen aus der Vielzahl von verschiedenen Winkeln transmittiert wird, durch jeden der Liniendetektoren aufgezeichnet wird, wobei die Bewegung über eine Länge ausgeführt wird, die jedem der Liniendetektoren das Scannen durch das gesamte Objekt ermöglicht, so dass für jeden der Liniendetektoren ein zweidimensionales Bild der Strahlung, die durch das Objekt hindurch in dem zugehörigen Winkel aus der Vielzahl von unterschiedlichen Winkeln transmittiert wird, erhalten wird.

15. Verfahren nach Anspruch 14, umfassend die weiteren Schritte:
- Drehen des Strahlungsdetektors (6) um einen Winkel (Δ) um eine Rotationsachse orthogonal zur Symmetrieachse (3), wobei die Liniendetektoren (6a), nach dieser Rotation, jeweils auf die Quelle divergenter Strahlung gerichtet sind, um einem Strahlenbündel dieser Strahlung, das in einem zugehörigen aus einer weiteren Vielzahl von unterschiedlichen Winkeln (α₁ + Δ, ..., αₙ + Δ, ..., α_{N} + Δ) propagiert zu ermöglichen, in den Liniendetektor einzufallen; und
- Wiederholen der im Wesentlichen linearen Bewegung der Quelle divergierender Strahlung und des Strahlungsdetektors relativ zu dem Objekt, während jeder der Liniendetektoren ausgelegt ist, eine weitere Vielzahl von Linienbildern der Strahlung, wie sie durch das Objekt in einem zugehörigen aus der weiteren Vielzahl von möglichen Winkeln (α₁ + Δ, ..., αₙ + Δ, ..., α_{N} + A) transmittiert wird, aufzuzeichnen.

16. Verfahren nach Anspruch 15, wobei die Rotationsachse durch die Quelle divergenter Strahlung (1) hindurchführt.

## Revendications

1. Appareil basé sur un balayage destiné à obtenir des données de tomosynthèse d'un objet (5) comprenant :
- une source de rayonnement divergent (1) destinée à émettre un rayonnement (2) centré autour d'un axe de symétrie (3) ;
- un détecteur de rayonnement (6) qui comprend une pile de détecteurs linéaires (6a), chacun d'eux étant dirigé vers la source de rayonnement divergent de telle sorte qu'un faisceau de rayons (b₁, ... , bₙ, ... , b_{N}) dudit rayonnement se propage sous l'un respectif d'une pluralité d'angles différents (α₁, ... , αₙ, ... , α_{N}) de manière à pénétrer dans le détecteur linéaire ;
- une zone d'objet disposée dans le chemin de rayonnement entre ladite source de rayonnement divergent et ledit détecteur de rayonnement, destinée à loger ledit objet ; et
- un dispositif (7) destiné à déplacer ladite source de rayonnement divergent et ledit détecteur par rapport audit objet d'une manière sensiblement linéaire dans une direction (8) sensiblement orthogonale audit axe de symétrie tandis que chacun des détecteurs linéaires (6a) enregistre une pluralité d'images linéaires du rayonnement émis à travers ledit objet sous ledit angle respectif de ladite pluralité d'angles différents, dans lequel :
- ledit dispositif (7) destiné à déplacer est apte à déplacer ladite source de rayonnement divergent et ledit détecteur de rayonnement par rapport audit objet d'une longueur qui permet le balayage de chacun desdits détecteurs linéaires à travers tout l'objet de manière à obtenir, pour chacun desdits détecteurs linéaires, une image bidimensionnelle du rayonnement émis à travers ledit objet sous ledit angle respectif de ladite pluralité d'angles différents.

2. Appareil selon la revendication 1, comprenant :
- un dispositif destiné à faire tourner ledit détecteur de rayonnement (6) d'un angle (Δ) autour d'un axe de rotation orthogonal audit axe de symétrie (3), les détecteurs linéaires (6a) étant chacun dirigés, après ladite rotation, vers la source de rayonnement divergent de manière à permettre au faisceau de rayons dudit rayonnement qui se propage sous l'angle respectif d'une autre pluralité d'angles différents angles (α₁ + Δ, ... , αₙ + Δ, ... , α_{N} + Δ), de pénétrer dans le détecteur linéaire, dans lequel
- ledit dispositif (7) destiné à déplacer est agencé en outre de manière à répéter le déplacement sensiblement linéaire de ladite source de rayonnement divergent et dudit détecteur de rayonnement par rapport audit objet, tandis que chacun desdits détecteurs linéaires est apte à enregistrer une autre pluralité d'images linéaires de rayonnement transmises à travers ledit objet sous l'angle respectif de ladite autre pluralité d'angles différents (α₁ + Δ, ... , αₙ + Δ, ... , α_{N} + Δ).

3. Appareil selon la revendication 2, dans lequel ledit axe de rotation passe à travers ladite source de rayonnement divergent (1).

4. Appareil selon la revendication 2 ou la revendication 3, dans lequel :
- ledit dispositif destiné à faire tourner est apte à faire tourner à plusieurs reprises ledit détecteur de rayonnement (6) autour dudit axe de rotation, les détecteurs linéaires (6a) étant chacun dirigés, après chacune desdites rotations, vers la source de rayonnement divergent de manière à permettre au faisceau de rayons dudit rayonnement qui se propage sous un angle respectif, de pénétrer dans le détecteur linéaire ; et
- ledit dispositif (7) destiné à déplacer est apte, après chacune desdites rotations, à répéter le déplacement sensiblement linéaire de ladite source de rayonnement divergent et dudit détecteur de rayonnement par rapport audit objet, tandis que chacun desdits détecteurs linéaires est apte à enregistrer lesdites images linéaires du rayonnement émises à travers ledit objet sous un dit angle respectif.

5. Appareil selon l'une quelconque des revendications 2 à 4, dans lequel lesdits détecteurs linéaires sont orientées de manière à détecter des images linéaires qui s'étendent dans une direction (y) sensiblement orthogonale audit axe de symétrie (3) et sensiblement orthogonale à la direction (8) afin de déplacer ladite source de rayonnement divergent et ledit détecteur de rayonnement par rapport audit objet.

6. Appareil selon la revendication 5, dans lequel ladite direction (y) dans laquelle lesdites images linéaires s'étendent, est parallèle audit axe de rotation.

7. Appareil selon la revendication 2 à 4, dans lequel :
- lesdits détecteurs linéaires sont orientées de manière à détecter des images linéaires qui s'étendent dans une direction (y) sensiblement orthogonale audit axe de symétrie (3) et sensiblement orthogonale à la direction (x) afin de déplacer ladite source de rayonnement divergent et ledit détecteur de rayonnement par rapport audit objet ; et
- ladite direction (y) dans laquelle lesdites images linéaires s'étendent, est sensiblement orthogonale audit axe de rotation (x).

8. Appareil selon l'une quelconque des revendications 1 à 7, dans lequel ladite pluralité d'angles différents est répartie dans une plage angulaire (α_{N} - α₁) au moins égale à 5°, de préférence au moins égale à 10°, et mieux encore au moins égale à 15°.

9. Appareil selon l'une quelconque des revendications 1 à 8, dans lequel le nombre de détecteurs linéaires (6a) dans ladite pile de détecteurs linéaires, est au moins égal à 3, de préférence au moins égal à 10, et mieux encore au moins égal à 25.

10. Appareil selon l'une quelconque des revendications 1 à 9, dans lequel :
- ladite source de rayonnement divergent est une source de rayons X (1) ; et
- lesdits détecteurs linéaires sont chacun un détecteur d'ionisation à base de gaz (6a), dans lesquels les électrons libérés en raison d'une ionisation par un faisceau de rayons respectif, sont accélérés dans une direction sensiblement perpendiculaire à la direction de ce faisceau de rayons.

11. Appareil selon la revendication 10, dans lequel ledit détecteur d'ionisation à base de gaz est un détecteur d'avalanche électronique (6a).

12. Appareil selon l'une quelconque des revendications 1 à 11, dans lequel lesdits détecteurs sont chacun l'un d'un réseau de diodes, d'un réseau à base de scintillateurs, d'un réseau CCD, d'un détecteur à base de TFT ou de CMOS ou d'un détecteur de liquide (6a).

13. Appareil selon l'une quelconque des revendications 1 à 12, comprenant un collimateur (4) disposé dans le chemin de rayonnement entre ladite source de rayonnement et ladite zone d'objet, ledit collimateur empêchant le rayonnement, qui n'est pas dirigé vers lesdits détecteurs linéaires, d'affecter ledit objet, en réduisant de ce fait la dose de rayonnement vers ledit objet.

14. Procédé basé sur un balayage destiné à obtenir des données de tomosynthèse d'un objet (5) comprenant les étapes suivantes consistant à :
- utiliser une source de rayonnement divergent (1) qui émet un rayonnement (2) centré autour d'un axe de symétrie (3) ; et un détecteur de rayonnement (6) qui comprend une pile de détecteurs linéaires (6a), chacun d'eux étant dirigé vers la source de rayonnement divergent de telle sorte qu'un faisceau de rayons (b₁, ... , bₙ, ..., b_{N}) dudit rayonnement se propage sous l'angle respectif d'une pluralité d'angles différents (α₁, ... , αₙ, ..., α_{N}) de manière à pénétrer dans le détecteur ;
- disposer ledit objet dans le chemin de rayonnement entre ladite source de rayonnement divergent et ledit détecteur de rayonnement ; et
- déplacer ladite source de rayonnement divergent et ledit détecteur de rayonnement par rapport audit objet de manière sensiblement linéaire dans une direction (8) sensiblement orthogonale audit axe de symétrie, tandis qu'une pluralité d'images linéaires de rayonnement transmises à travers ledit objet sous ledit angle respectif de ladite pluralité d'angles différents, sont enregistrés par chacun desdits détecteurs linéaires, dans lequel ledit déplacement est exécuté d'une longueur qui permet le balayage de chacun desdits détecteurs linéaires à travers tout l'objet de manière à obtenir, pour chacun desdits détecteurs linéaires, une image bidimensionnelle du rayonnement transmis à travers ledit objet sous ledit un respectif de ladite pluralité d'angles différents.

15. A procédé selon la revendication 14, comprenant les étapes supplémentaires consistant à :
- faire tourner ledit détecteur de rayonnement (6) d'un angle (Δ) autour d'un axe de rotation orthogonal audit axe de symétrie (3), les détecteurs linéaires (6a) étant chacun dirigés, après ladite rotation, vers la source de rayonnement divergent de manière à permettre au faisceau de rayons dudit rayonnement qui se propage sous l'angle respectif d'une autre pluralité d'angles différents angles (α₁ + Δ, ... , αₙ + Δ, ... , α_{N} + Δ), de pénétrer dans le détecteur linéaire ; et
- répéter le déplacement sensiblement linéaire de ladite source de rayonnement divergent et dudit détecteur de rayonnement par rapport audit objet, tandis que chacun desdits détecteurs linéaires est apte à enregistrer une autre pluralité d'images linéaires émises à travers ledit objet sous l'angle respectif de ladite autre pluralité d'angles différents (α₁ + Δ, ... , αₙ + Δ, ... , α_{N} + Δ).

16. Procédé selon la revendication 15, dans lequel ledit axe de rotation passe à travers ladite source de rayonnement divergent (1).
